# EUROPEAN PATENT SPECIFICATION

(11) **EP 3 030 672 B1**
(45) Date of publication and mention of the grant of the patent: **02.08.2017**
(21) Application number: 14747630.3
(22) Date of filing: 01.08.2014
(51) Int. Cl.: C12Q 1/68

(54) **ANALYSIS FOR TAKOTSUBO CARDIOMYOPATHY BY MEANS OF DIFFERENTIALLY REGULATED MICRORNA MOLECULES**
ANALYSE FÜR TAKOTSUBO-KARDIOMYOPATHIE MITTELS DIFFERENTIELL REGULIERTER MIKRO-RNA-MOLEKÜLE
ANALYSE D'UNE CARDIOMYOPATHIE DE TAKO-TSUBO AU MOYEN DE MOLÉCULES DE MICRO-ARN DIFFÉRENTIELLEMENT RÉGULÉS DIFFÉREMMENT

(30) Priority: 06.08.2013 EP 13179470
(43) Date of publication of application: 15.06.2016
(73) Proprietor: Medizinische Hochschule Hannover, 30625 Hannover (DE)
(72) Inventor: THUM, Thomas, 30675 Hannover (DE); TEMPLIN, Christian, CH-8091 Zürich (CH); VOLKMANN, Julia, 30625 Hannover (DE)
(74) Representative: Taruttis, Stefan Georg
(86) International application number: PCT/EP2014/066669
(87) International publication number: WO 2015/018771

(56) References cited:
- WO-A2-2008/042231
- WO-A2-2012/160551
- KUWABARA YASUHIDE ET AL: "Increased microRNA-1 and microRNA-133a levels in serum of patients with cardiovascular disease indicate myocardial damage.", CIRCULATION. CARDIOVASCULAR GENETICS 1 AUG 2011, vol. 4, no. 4, 1 August 2011 (2011-08-01), pages 446-454, XP002732481, ISSN: 1942-3268
- KUWABARA Y ET AL: "Elevated microRNA-1 and microRNA-133a levels in serum of patients with cardiovascular diseases indicate the myocardial damage", EUROPEAN HEART JOURNAL, vol. 32, no. Suppl. 1, August 2011 (2011-08), page 392, XP002732482, & CONGRESS OF THE EUROPEAN-SOCIETY-OF-CARDIOLOGY; PARIS, FRANCE; AUGUST 27 -31, 2011
- CHUANWEI LI ET AL: "Circulating microRNAs as novel and sensitive biomarkers of acute myocardial Infarction", CLINICAL BIOCHEMISTRY, ELSEVIER INC, US, CA, vol. 45, no. 10, 9 April 2012 (2012-04-09) , pages 727-732, XP028494366, ISSN: 0009-9120, DOI: 10.1016/J.CLINBIOCHEM.2012.04.013 [retrieved on 2012-04-26]
- YILDIRIM SAMET SERDAR ET AL: "Relationship Between Downregulation of miRNAs and Increase of Oxidative Stress in the Development of Diabetic Cardiac Dysfunction: Junctin as a Target Protein of miR-1", CELL BIOCHEMISTRY AND BIOPHYSICS, vol. 67, no. 3, 31 May 2013 (2013-05-31), pages 1397-1408, XP002732483,
- CHEN YAN ET AL: "Circulating microRNAs, novel biomarkers of acute myocardial infarction: a systemic review.", WORLD JOURNAL OF EMERGENCY MEDICINE 2012, vol. 3, no. 4, 2012, pages 257-260, XP002732484, ISSN: 1920-8642

### SEQUENCE LISTING

<110> Medizinische Hochschule Hannover UniversitÃ¤t ZÃ¼rich
<120> Analysis for Takotsubo cardiomyopathy
<130> M1054PCT
<150> EP13179470
   <151> 2013-08-06
<160> 8
<170> BiSSAP 1.2
<210> 1
   <211> 22
   <212> RNA
   <213> Homo sapiens
<220>
   <221> source
   <222> 1..22
   <223> /mol_type="other RNA" /note="microRNA miR-16" /note="ncRNA class = siRNA" /organism="Homo sapiens"
<400> 1
   uagcagcacg uaaauauugg cg 22
<210> 2
   <211> 22
   <212> RNA
   <213> Homo sapiens
<220>
   <221> source
   <222> 1..22
   <223> /mol_type="other RNA" /note="microRNA miR-26a" /note="ncRNA class = siRNA" /organism="Homo sapiens"
<400> 2
   uucaaguaau ccaggauagg cu 22
<210> 3
   <211> 22
   <212> RNA
   <213> Homo sapiens
<220>
   <221> source
   <222> 1..22
   <223> /mol_type="other RNA" /note="microRNA miR-1" /note="ncRNA class = siRNA" /organism="Homo sapiens"
<400> 3
   uggaauguaa agaaguaugu au 22
<210> 4
   <211> 22
   <212> RNA
   <213> Homo sapiens
<220>
   <221> source
   <222> 1..22
   <223> /mol_type="other RNA" /note="microRNA miR-133a" /note="ncRNA class = siRNA" /organism="Homo sapiens"
<400> 4
   uuuggucccc uucaaccagc ug 22
<210> 5
   <211> 22
   <212> RNA
   <213> Homo sapiens
<220>
   <221> source
   <222> 1..22
   <223> /mol_type="other RNA" /note="microRNA miR-letf7" /note="ncRNA class = siRNA" /organism="Homo sapiens"
<400> 5
   ugagguagua gauuguauag uu 22
<210> 6
   <211> 21
   <212> RNA
   <213> Homo sapiens
<220>
   <221> source
   <222> 1..21
   <223> /mol_type="other RNA" /note="microRNA miR-410" /note="ncRNA class = siRNA" /organism="Homo sapiens"
<400> 6
   aauauaacac agauggccug u 21
<210> 7
   <211> 21
   <212> RNA
   <213> Homo sapiens
<220>
   <221> source
   <222> 1..21
   <223> /mol_type="other RNA" /note="microRNA miR-640" /note="ncRNA class = siRNA" /organism="Homo sapiens"
<400> 7
   augauccagg aaccugccuc u 21
<210> 8
   <211> 22
   <212> RNA
   <213> Caenorhabditis elegans
<220>
   <221> source
   <222> 1..22
   <223> /mol_type="other RNA" /note="microRNA cel-miR39" /note="ncRNA class = siRNA" /organism="Caenorhabditis elegans"
<400> 8
   ucaccgggug uaaaucagcu ug 22

## Description

The present invention relates to an analytical method for the analysis of Takotsubocardiomyopathy (TTC) in a patient sample, especially for differentiating the TTC from acute myocardial infarction. Accordingly, the method can determine in a sample from a patient analytes specific for cardiomyopathy as well as analytes specific for TTC. Preferably, the method in a sample determines analytes specific for TTC and differentiates TTC from myocardial infarction. Further, the invention relates to the use of compounds as probes in the analytical method, especially for analysing a sample for TTC, and to the use of a kit of parts for the analytical method.

The present invention is based on the finding that specific analytes indicate the occurence of TTC in a patient, especially differentiating the occurrence of TTC from the occurrence of acute myocardial infarction.

### State of the art

TTC as referred to in the present invention, has first been described by Sato et al. (Clinical aspect of myocardial injury: from ischemia to heart failure, Heart Fail. 1990: 56 - 64) as a transient, reversible, regional systolic dysfunction of the left ventricle. In TTC patients, there often is hypokinesis or akinesis of the apical region despite any obstructive epicardial coronary artery disease, while the basal segments of the left ventricle remain in a hypercontractile state. It is known, e.g. from Dote et al., (Myocardial stunning due to simultaneous multivessel coronary spasms: a review of 5 cases, J. Cardiol. 1991; 21: 203 - 214) that the clinical symptoms and findings of TTC can mimic those of acute myocardial infarction, e.g. in respect of acute onset of angina, as T - segment elevation or depression noted on the ECG, and arise in cardiac enzyme levels above normal limit.

WO 2012/160551 A2 describes that in heart failure patients hsa-miR-26a, hsa-miR-199b-5p, hsa-miR-33a and hsa-miR-27b are significantly downregulated in comparison to levels in healthy persons.

WO 2008/042231 A3 describes a test for determination of the type of heart disease in a patient, comprising analysis of a myocard sample for expression of a number of microRNAs including miR-13 3 a, miR-16 and miR-1.

Kuwabara et al., Circ.Cardiovasc. Genet.4, 4, p. 446-454 (2011) describes increased serum levels of miR-1 and of miR-133a in patients not only with myocardial infarction but also with unstable angina pectoris and TTC without elevation of serum creatine phosphokinase or cardiac troponin.

Kuwabara et al., European Heart Journal 2011, page 392 (D4) describe that elevated levels of miR-1 and miR-133a in serum of patients with cardiovascular diseases indicate myocardial damage.

### Object of the invention

It is an object of the invention to provide an analytical method for identifying the presence of TTC in patients, especially for differentiating TTC from acute myocardial infarction in patients.

### General description of the invention

The invention achieves the object by providing an analytical method according to claim 1 suitable for detecting the presence of TTC, especially for differentiating TTC from myocardial infarction, especially from STEMI, by analysing a sample obtained from a patient, preferably a blood sample, especially a serum sample for the concentration (level) of least one of the following microRNAs (miR): miR-16 (UAGCAGCACGUAAAUAUUGGCG, SEQ ID NO: 1), miR-26a (UUCAAGUAAUCCAGGAUAGGCU, SEQ ID NO: 2), preferably of miR1 (UGGAAUGUAAAGAAGUAUGUAU, SEQ ID NO: 3) and/or of miR133a (UUUGGUCCCCUUCAACCAGCUG, SEQ ID NO: 4), optionally in addition of miR-let7f (UGAGGUAGUAGAUUGUAUAGUU, SEQ ID NO: 5), of miR- 410 (AAUAUAACACAGAUGGCCUGU, SEQ ID NO: 6), and/or of miR-640 (AUGAUCCAGGAACCUGCCUCU, SEQ ID NO: 7). The concentration of the at least one microRNA is determined, e.g. by analysing the microRNA and determining its concentration in relation to the concentration of a reference or standard nucleic acid, e.g. a different microRNA, or in relation to the concentration of the respective microRNA (miR) in a sample of a person not suffering from myocardial infarction, e.g. from a healthy person, and/or by adding to the sample a known concentration of a microRNA serving as an internal standard, e.g. a human or a non-human microRNA, or the same microRNA. Examples for a different microRNA suitable as a standard nucleic acid are C. elegans cel-miR39 (UCACCGGGUGUAAAUCAGCUUG, SEQ ID NO: 8). The concentration of the microRNA can e.g. be analysed using quantitative RT-PCR or using hybridization to a specific nucleic acid probe molecule with quantitative detection. In the nucleic acid sequence protocol, the sequences of mature microRNAs are given.

It has been found that an elevated level of each of miR-16 and of miR-26a indicates presence of TTC, also in presence of elevated levels of miR1 and of miR133a, but absence of acute myocardial infarction, especially of acute ST elevation myocardial infarction (STEMI). Elevated levels of miR1 and of miR133a are found in both samples from STEMI patients and in samples from TTC patients, with generally higher elevation levels in STEMI patients compared to TTC patients.

The elevation of levels of miR16 and of miR26a in samples from TTC patients has been found to be significant over both samples from healthy persons and samples from STEMI patients, with levels of miR16 and of miR26a generally being similar in healthy persons and STEMI patients.

Preferably, the method includes analysis of the concentration of miR-133a and/or of miR-1 in the sample, because a low concentration of miR-133a and/or of miR-1 indicates TTC, whereas a higher concentration of miR-113a and/or of miR-1 indicates presence of STEMI in patients, or absence of TTC cardiomyophathy.

As a further indicator, the miR let-7f can be analyzed in the process, with a level of miR let-7f that is elevated in comparison to the level in samples from myocardial infarction patients indicating TTC rather than STEMI.

Further, the invention relates to the use of nucleic acid molecules as probes in the process, for detecting the concentration of at least one of miR-16, miR-26a, preferably for detecting the concentration of miR-16 and of miR-26a in combination with miR-410 and miR-640 and/or optionally miR let-7f, in a sample, especially for detecting an elevated concentration of the least one of these, preferably in combination with the use of nucleic acid molecules as probes in the process, which are specific for at least one of miR-133a and miR-1, especially for detecting a non-elevated concentration thereof, e.g. a concentration corresponding to the concentration found in healthy persons.

Such nucleic acid molecules can be selected from primer pairs suitable for amplifying the respective microRNA, or from nucleic acid molecules hybridising specifically to the microRNA, e.g. nucleic acid molecules having the reverse complementary sequence to the microRNA to be analyzed.

It is an advantage of the present invention that the analytical method provides for data that can be used for diagnosing the presence of TTC, while eliminating acute myocardial infarction from the diagnosis without the need for invasive analysis, like e.g. catheterisation.

A kit of parts adapted to the analytical method of the invention contains nucleic acid molecules suitable as probes for detecting the at least one microRNA, e.g. specific primer pairs and/or reverse complementary nucleic acid molecules. Optionally, the kit of parts further contains a nucleic acid molecule for use as an internal reference, e.g. a non-human microRNA, and nucleic acid molecules fur use as probes for detecting the internal reference molecule, e.g. specific primer pairs and/or reverse complementary nucleic acid molecules. Accordingly, the analytical method preferably includes the step of adding to a sample a known amount of standard as a dopant, wherein the dopant forms an internal standard and wherein the standard preferably has the form of an RNA, preferably a microRNA, which RNA or microRNA, respectively can comprise or consist of a non-human RNA, e.g. cel-miR-39, and/or which RNA or microRNA, respectively can comprise or consist of the sequence of the analyte, i.e. miR-16, miR-26a, miR-1, miR-133a, miR let-7f, miR-410 and/or miR-640 or combinations of two or more of these. In these embodiments, an aliquot of the sample may be doped with a standard in the form of a RNA and another aliquot may be subjected to the analytical method without addition of a standard. Generally, it is preferred in these embodiments that the detected level of the microRNA is reduced by the amount detected on the basis of the standard added to the sample. Therein, the amount detected on the basis of the standard is the level that is detected for the added standard in addition to the original analyte content of the probe.

Optionally, the analytical method comprises or consists of detecting the level of the analytes miR-16, miR-26a, miR-1 and miR-133a, and optionally further including miR let-7f, miR-410 and/or miR-640 in a sample, and comparing the levels detected for each microRNA with the level of the respective microRNA determined in a sample from a STEMI patient and/or with the level of the respective microRNA determined in a sample from a healthy person. The level of the respective microRNA determined in a sample from a STEMI patient and/or the level of the respective microRNA determined in a sample from a healthy person can be predetermined. These embodiments can be performed with or without adding a known amount of standard RNA or microRNA as a dopant, and when adding the dopant, optionally reducing the level of detected analyte by the amount detected on the basis of the added standard (dopant).

It has been found that in the majority of infarction patients diagnosed with TTC, the level of miR-16 and the level of miR-26a significantly decreases from a first to a second sampling point in time. Therefore, the analytical method preferably comprises detecting the level of the analyte, preferably of miR-16 and/or of miR-26a in a sample obtained at a first sampling point in time and in a sample obtained at a second sampling point in time, wherein the second sampling point in time is at least 10 h to 24 h later than the first sampling point in time. A sampling point in time is the time at which the sample is taken from the patient.

Optionally, the analytical method can indicate TTC, preferably in combination with indicating absence of STEMI, in a sample obtained from an infarction patient by a significant increase in the level of miR-16, e.g. by a factor of at least 3 in comparison to the level of the same miR in a sample from a healthy person and/or in comparison to the level of the same miR in a in a sample from a STEMI patient, and/or by a significant increase in the level of miR-26a, e.g. by factor of at least 5 or 6 in comparison to the level of the same miR in a in a sample from a healthy person and/or in comparison to the level of the same miR in a in a sample from a STEMI patient.

Optionally additionally, the analyical methodn indicate TTC, preferably in combination with indicating absence of STEMI, in a sample obtained from an infarction patient by an increase of the concentration of miR-1, e.g. by factor of at maximum 2 or 2.5 in comparison to the level of the same miR in a in a sample from a healthy person and/or by a factor of approx. 2 to 3 in comparison to the level of the same miR in a in a sample from a STEMI patient, of miR-133a, e.g. by factor of at maximum 2 to 4 in comparison to the level of the same miR in a in a sample from a healthy person,
of miR-410, e.g. by factor of at approx. 3 in comparison to the level of the same miR in a in a sample from a healthy person,
of miR-640 by factor of approx. 1.5 in comparison to the level of the same miR in a in a sample from a healthy person, and/or
of miR let-7f by factor of at least 1.5 or at least 2 or at least 3 in comparison to the level of the same miR in a in a sample from a healthy person and/or in comparison to the level of the same miR in a in a sample from a STEMI patient. Optionally, the level of each miR is determined in relation to an internal standard which e.g. is an added known amount of an RNA, preferably a microRNA, of a non-human or a human nucleic acid sequence as described herein.

For miR-133a, an increase of concentration by a factor of at least 30 in comparison to the level of the same miR in a in a sample from a healthy person from has been determined in samples from STEMI patients. Accordingly, an increase of the concentration of miR-133a by a factor of at least 20 to 30 in comparison to the concentration in a sample from a healthy person is indicative for STEMI, whereas a concentration of miR-133a increased by a factor of at maximum 4 in comparison to the concentration in a sample from a healthy person is indicative of TTC.

For miR-1, an more important increase of concentration, e.g. by a factor of approx. 10 in comparison to the level of the same miR in a in a sample from a healthy person from has been determined in samples from STEMI patients. Accordingly, an increase of the concentration of miR-1 by a factor of at least 9, preferably of at least 10, in comparison to the concentration in a sample from a healthy person is indicative for STEMI, whereas a less important increase in the concentration of miR-1, e.g. increased by a factor of at maximum 2.5, preferably of at maxmum 3, in comparison to the concentration in a sample from a healthy person is indicative of TTC.

### Detailed description of the invention

The invention is now described in greater detail by way of examples with reference to the figures which show
- in Figure 1 the relative concentration of miR-16 in samples from healthy persons, from STEMI patients and from TTC - patients,
- in Figure 2 the relative concentration of miR-26a in healthy persons, in STEMI - patients and in TTC - patients,
- in Figure 3 A) relative concentrations of miR-16 in patients suffering from depression and being free from depression, resp., and in B) relative concentrations of miR- 26a in patients suffering from depression and being free from depression, in C) the concentration of miR-16 in the presence or absence of SSRI, and in D) the concentration of miR-26a in patients with and without administration of antidepressant drugs,
- in Figure 4 the concentration of miR-410 in healthy persons and in TTC patients,
- in Figure 5 the relative concentration of miR-640 in healthy persons and in TTC patients,
- in Figure 6 the concentration of miR-133a in healthy persons, in STEMI - patients and in TTC - patients,
- in Figure 7 the concentration of miR-1 in healthy persons, in STEMI - patients, and in TTC - patients,
- in Figure 8 the concentration of miR let-7f in healthy persons, in STEMI - patients, and in TTC - patients,
- in Figure 9 A to E the sensitivity for the specificity of the microRNA indicated for detecting TTC, once in respect of STEMI and once in respect of healthy persons,
- Figure 10 the concentrations of miR-16 in TTC patient samples at a first point in time 1 and at a later second point in time 2 for different patients, and
- Figure 11 the concentrations of miR-26a in TTC patient samples at a first point in time 1 and at a later second point in time 2 for different patients.

In the Figures, * denotes p<0.05, ** denotes p<0.01, and *** denotes p<0.001, n.s. denotes no significance between values.

### Example: Analysis of blood plasma samples from infarction patients

Blood plasma samples were collected from 33 TTC - patients, from 28 patients with acute ST elevation myocardial infarction (STEMI), and from 28 healthy persons serving as a control. Blood samples were collected within 24 hours from the onset of chest pain.

RNA was isolated from 100 µl plasma with the MiRNeasy Isolation Kit (available from Qiagen, Hilden, Germany), according to the manufacturer's instructions, after adding 5 µl of 1 fmol/µl cel-miR-39 (miR-39 of Caenorhabditis elegans) (SEQ ID NO: 8) as an internal standard before starting the isolation procedure.

MicroRNAs were analysed using reverse transcription - polymerase chain reaction (RT-PCR). Preferably, quantitative RT-PCR was made using the TaqMan microRNA Reverse Transcription Kit (available from Applied Biosystems) using labelled primers, e.g. one primer of a pair was labelled with a fluorochrome, and the other primer of the pair was labelled with a quencher specific for the emission of the fluorochrome. Results were normalized to the amount of the added cel-miR- 39, serving as an internal standard.

Results are given as mean values +/- SEM. Differences between groups were analysed by the student t-test, Excel, or by one-way ANOVA, followed by Bonferroni's Multiple Comparison Test, applied as a post-hoc test.

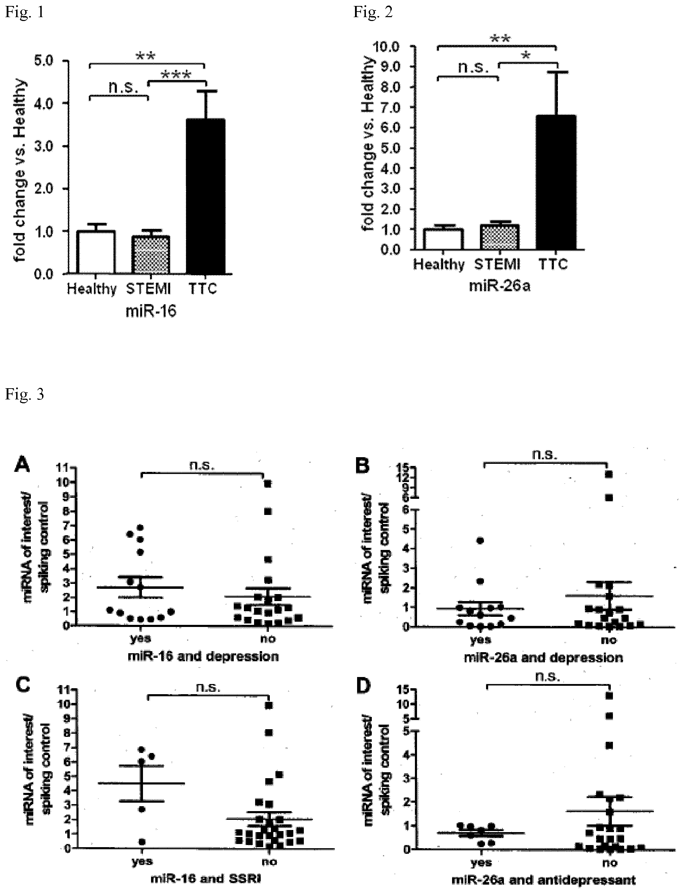

Figure 1 shows the relative concentration of miR-16 in healthy persons, in STEMI - patients, and in TTC - patients, respectively. It can be seen that the concentration of miR-16 is significantly elevated specifically in TTC - patients, approximately a factor of at least 3 over the concentration in healthy persons and/or in STEMI - patients.

Figure 2 shows that the relative concentration of miR-26a is significantly elevated in TTC - patients, e.g. by a factor of about 6, over the concentration of miR-26a in healthy persons or in STEMI - patients.

These data show that an elevated concentration of each of miR-16 and miR-26a specifically indicates TTC by excluding STEMI or the absence of an infarction (healthy persons).
As miR-16 and miR-26a are also known to be correlated with depression, the correlation with depression, exemplified by SSRI, and administration of antidepressant were analyzed.

Figure 3A) shows that the concentration of the miR-16 does not significantly differ in samples obtained from TTC - patients who are free from a depression (no), and in patients suffering from a depression (yes). Correspondingly, Figure 3B) shows the concentration of miR-26a in patients being free from a depression (no), and in patients suffering from a depression (yes). The data show that concentrations of miR-26a do not significantly differ in TTC - patients suffering from or being free from a depression.

Accordingly, Figures 3A) and 3B) show that an elevated concentration of each of miR-16 and miR-26a independent from presence or absence of a depression in the patient specifically indicates TTC, e.g. excluding presence of STEMI.

Figure 3C) shows the relative concentration of miR-16 in the absence of (SSRI) (no) and in the presence of SSRI (yes) in TTC - patients. These data show that the elevated relative concentration of miR-16 independently from SSRI indicates TTC, e.g. eliminating the presence of STEMI.

Figure 3D) shows the relative concentration of miR-26a in the absence of antidepressant drugs from TTC - patients (no), and with application of antidepressant (yes) in TTC - patients. These data show that there is no significant influence of the presence of antidepressant on the specificity of the elevated concentration of miR-26a in TTC - patients.

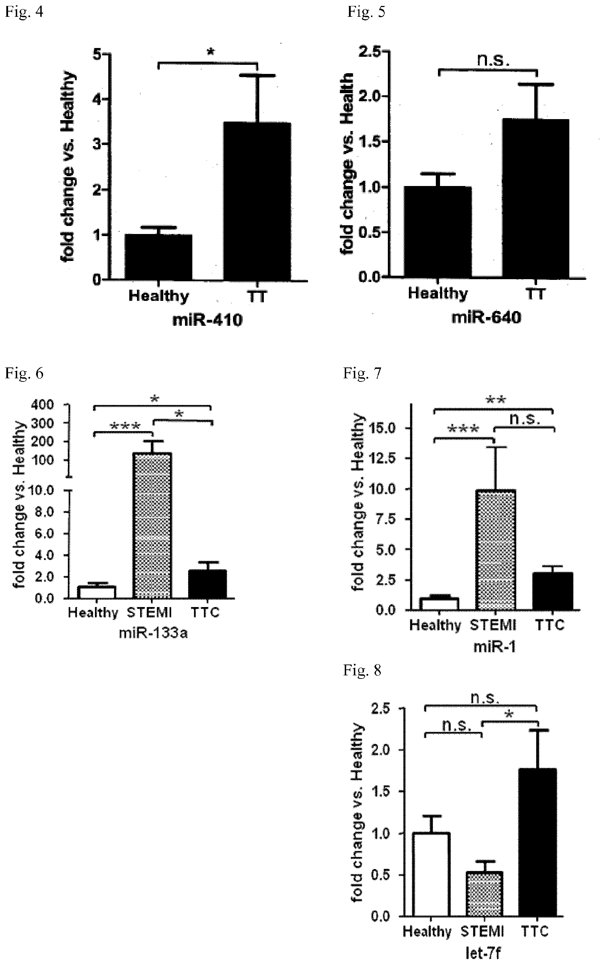

Figure 4 shows the relative concentration of miR-410 in TTC - patients and in healthy persons. The concentration of miR-410 is increased by a factor of approximately 3 in TTC - patients.

Figure 5 shows the elevated concentration of miR-640 in TTC - patients by a factor of approximately 1.7 in comparison to healthy persons.

The data shown in Figures 4 and 5 show that an elevated concentration of miR-410 and/or of miR-640 also indicates presence of TTC.

Figure 6 shows the relative concentration of miR-133a and makes it clear that the concentration of miR-133a is not significantly elevated in TTC - patients over the concentration in healthy persons, whereas the concentration of miR-133a is significantly increased, e.g. by a factor of more than 100, in STEMI - patients compared to healthy persons and also compared to TTC - patients. This shows that elevated serum levels of miR-133a rather indicate STEMI but are not significantly indicative of TTC.

Figure 7 shows that the elevated concentration of miR-1 is indicative of the presence of STEMI, e.g. by a factor of approximately 10 over the concentration found in healthy persons. The elevated concentration of miR-1 in TTC - patients is not significant.

The data shown in Figures 6 and 7 show that the concentrations of miR-113a and of miR-1 are not significantly increased in TTC - patients compared to healthy persons, but that a significant increase in the concentration of miR-133a or of miR-1 over the concentration found in healthy persons indicates the presence of STEMI and the absence of TTC.

Figure 8 shows that a concentration of the miR let-7f is significantly increased in TTC - patients, whereas its concentration can be decreased in STEMI - patients, showing that an increase of the concentration of miR let-7f is indicative of TTC rather than of STEMI.

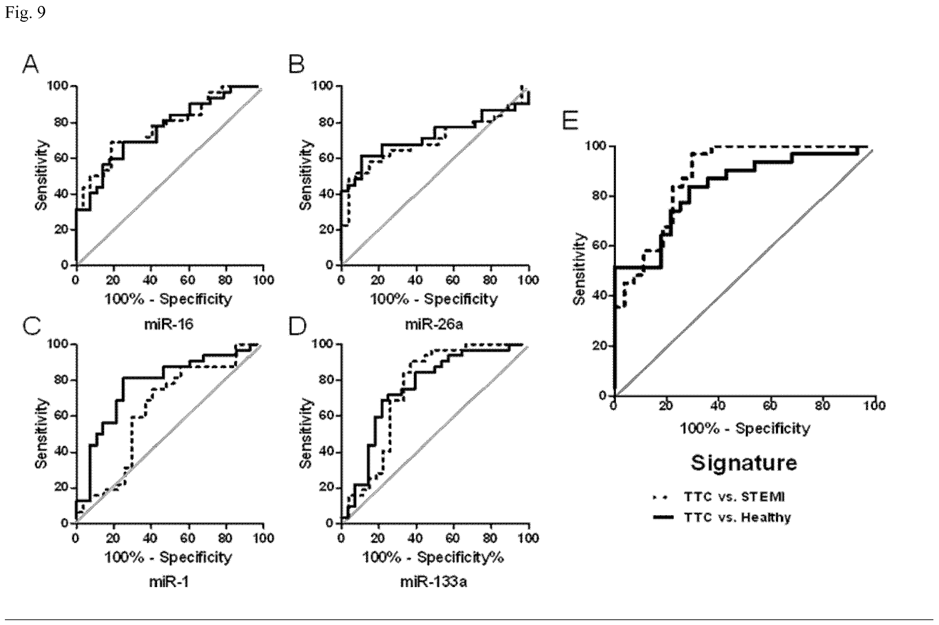

Figure 9 shows the correlation of specificity and sensitivity of the concentration A) of miR-16, B) miR-26a, C) miR-1, D) miR-133a, and at E) for combined miR-16, miR-26a, miR-1 and miR-133a, each in respect of TTC. This mathematical analysis was done using ROC analysis and shows the high specificity and sensitivity of the analytical method when analyzing the concentration of each of these analytes singly, and especially of the analytical method when analyzing the combination of the analytes miR-16, miR-26a, miR-1 and miR-133a.

In addition to the analytical data depicted in Figures 1 to 9, Figure 10 shows concentrations determined by the analytical method at a first point in time (1) and at a second point in time (2), which was 10 h to 24 h later in the same TTC patient. Samples from the same patient are indicated for each graph as TT-55, TT-49, TT-52, TT-53, TT-46, TT-56, TT-38, and TT-50, respectively. Except for the samples from patient TT-50, the analytical method determines a significant decrease in concentration of the analyte miR-16 from the first point in time (1) to the later second timpoint (2). At present, it cannot be ruled out that the data for patient TT-50 have been mixed up.

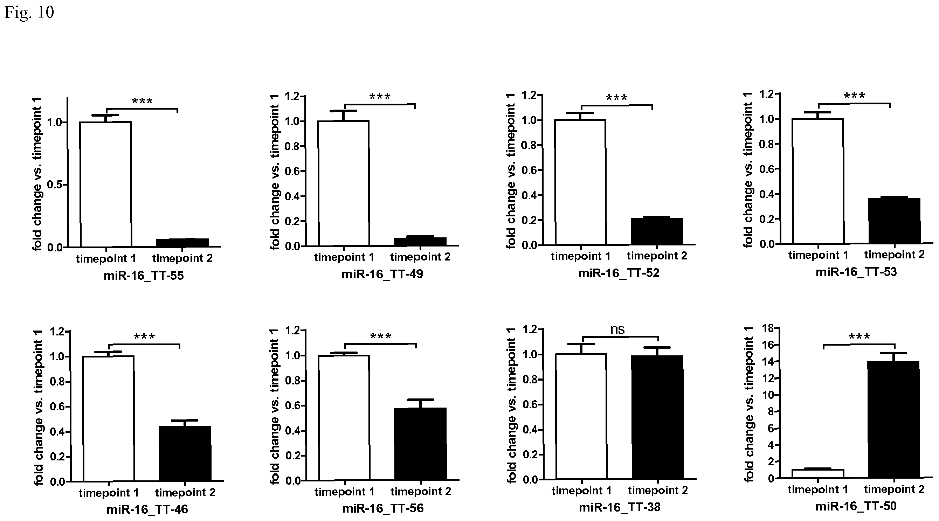

Generally, the data of Fig. 10 show that an analytical method analyzing a sample taken at a later second point in time from the same patient in addition to analyzing the sample taken at the first point in time confirm the presence of TTC when the concentration of miR-16 remains or preferably is reduced.

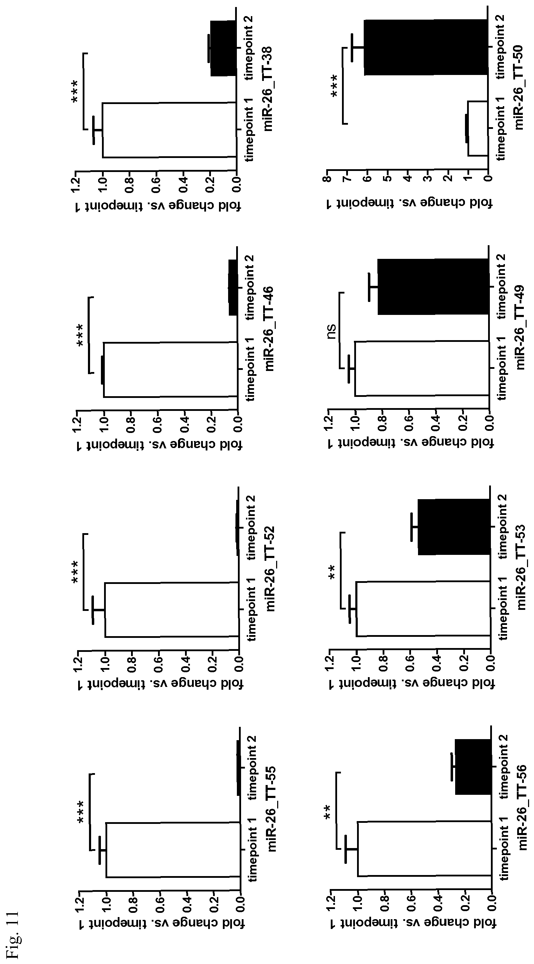

Figure 11 shows the concentrations of miR-26a for the first point in time (1) and the second point in time (2) for the samples as analyzed for Fig. 10. Again with the exception of the samples of patient TT-50, the results show that an analytical method analyzing a sample taken at a later, second point in time from the same patient in addition to analyzing the sample taken at the first point in time confirm the presence of TTC when the concentration of miR-26a remains or preferably is reduced.

## Claims

1. Analytical method for detecting Takotsubo cardiomyopathy (TTC) in a patient sample by determining the concentration of microRNA in the nucleic acids of the sample, which microRNA is selected from the group comprising miR-16 (SEQ ID NO: 1) and miR-26a (SEQ ID NO: 2), **characterized in that** the concentration of the microRNA selected from miR-16 (SEQ ID NO: 1) and miR-26a (SEQ ID NO: 2) is detected as significantly elevated in comparison to the concentration of the microRNA determined in a sample from a healthy person and/or in comparison to the concentration of the microRNA determined in a sample from a STEMI patient.

2. Analytical method according to claim 1, **characterized in that** additionally the concentration of at least one microRNA from the group consisting of miR-1 (SEQ ID NO: 3) and/or of miR-133a (SEQ ID NO: 4) is determined.

3. Analytical method according to one of the preceding claims, **characterized in that** additionally the concentration of at least one microRNA from the group consisting of miR let-7f (SEQ ID NO: 5), miR-410 (SEQ ID NO: 6) and/or of miR-640 (SEQ ID NO: 7) is determined.

4. Analytical method according to one of the preceding claims, **characterized in that** the concentration is determined by quantitative reverse transcription polymerase chain reaction using primer pairs specific for amplification of the microRNA.

5. Analytical method according to one of the preceding claims, **characterized in that** the concentration is determined by detecting hybridisation using a nucleic acid molecule comprising a sequence that is reverse complementary to the microRNA.

6. Analytical method according to one of the preceding claims, **characterized by** adding to the sample a known amount of a non-human microRNA prior to determining the concentration of the microRNA, determining the concentration of the non-human microRNA, and calculating the concentration of the microRNA in relation to the amount of the added non-human microRNA.

7. Analytical method according to one of the preceding claims, **characterized in that** the sample is a blood sample, preferably a serum sample.

8. Analytical method according to one of claims 2 to 7, **characterized in that** a concentration of miR-1 determined as increased by a factor of approximately 2 to 3 in comparison to the concentration of miR-1 in a sample from a healthy person is indicative for TTC, and/or that a concentration of miR-133a determined as increased by a factor of at maximum 2 to 4 in comparison to the concentration of miR-133a in a sample from a healthy person is indicative of TTC, and/or that a concentration of the microRNA miR-410 (SEQ ID NO: 3) determined as increased by a factor of approximately 3 in comparison to the concentration in a sample from a healthy person is indicative of TTC, and/or that a concentration of miR-640 (SEQ ID NO: 4) determined as increased by a factor of approximately 1.5 in comparison to the concentration in a sample from healthy person is indicative of TTC.

9. Analytical method according to claim 8, **characterized in that** the increased concentrations of miR-1, miR-133a, miR-410 and/or of miR-640 are indicative of the absence of STEMI.

10. Analytical method according to one of the preceding claims, **characterized in that** the concentration of the microRNA is determined in one sample taken at a first point in time and in one sample taken at a later second point in time from the same patient.

11. Analytical method according to claim 10, **characterized in that** a concentration of miR-26a and/or of miR-16 which is decreased in the sample taken at the second point in time in comparison to the concentration in the sample taken at the first point in time is indicative of TTC.

12. Analytical method according to one of the preceding claims, **characterized by** adding to the sample a known amount of a microRNA selected from the group consisting of miR-16 (SEQ ID NO: 1) and miR-26a (SEQ ID NO: 2) prior to determining the concentration of the microRNA and when determining the concentration of the microRNA, reducing the concentration of the microRNA by the amount that was predetermined to be generated from the added microRNA.

13. Analytical method according to one of claims 6 to 12, **characterized by** adding the known amount of a non-human microRNA and/or a microRNA selected from the group consisting of miR-16 (SEQ ID NO: 1) and miR-26a (SEQ ID NO: 2) prior to isolating RNA from the sample.

14. Use of a kit of parts in a method according to one of the preceding claims for analysing a patient sample for detecting an analyte indicative for Takotsubo cardiomyopathy (TTC) in the patient sample, the kit comprising a pair of primers which are at least in a terminal section reverse complementary to one of the microRNAs of the group consisting of miR-16 (SEQ ID NO: 1) and miR-26a (SEQ ID NO: 2).

15. Use according to claim 14, the kit further comprising an RNA comprising or consisting of miR-16 (SEQ ID NO: 1) and miR-26a (SEQ ID NO: 2) for use as an added standard to the sample.

## Patentansprüche

1. Analytisches Verfahren zum Nachweisen der Takotsubo Kardiomyopathie (TTC) in einer Patientenprobe durch Bestimmen der Konzentration von microRNA in den Nukleinsäuren der Probe, wobei die microRNA aus der Gruppe ausgewählt ist, die miR-16 (SEQ ID NO: 1) und miR-26a (SEQ ID NO: 2) umfasst, **dadurch gekennzeichnet, dass** die Konzentration der microRNA, die aus miR-16 (SEQ ID NO: 1) und miR-26 (SEQ ID NO: 2) ausgewählt ist, als signifikant erhöht im Vergleich zu Konzentration der microRNA ist, die in einer Probe von einer gesunden Person und/oder im Vergleich zur Konzentration der microRNA in einer Probe aus einem STEMI-Patienten bestimmt wird.

2. Analytisches Verfahren nach Anspruch 1, **dadurch gekennzeichnet, dass** zusätzlich die Konzentration wenigstens einer microRNA aus der Gruppe bestimmt wird, die aus miR-1 (SEQ ID NO: 3) und/oder miR-133a (SEQ ID NO: 4) besteht.

3. Analytisches Verfahren nach einem der voranstehenden Ansprüche, **dadurch gekennzeichnet, dass** zusätzlich die Konzentration von wenigstens einer microRNA aus der Gruppe bestimmt wird, die aus miR let-7f (SEQ ID NO: 5), miR-410 (SEQ ID NO: 6) und/oder miR-640 (SEQ ID NO: 7) besteht.

4. Analytisches Verfahren nach einem der voranstehenden Ansprüche, **dadurch gekennzeichnet, dass** die Konzentration durch quantitative revers transkribierende Polymerase-Ketten-Reaktion unter Verwendung von Primerpaaren bestimmt wird, die spezifisch für die Amplifikation der microRNA sind.

5. Analytisches Verfahren nach einem der voranstehenden Ansprüche, **dadurch gekennzeichnet, dass** die Konzentration durch Detektieren der Hybridisation mit einem Nukleinsäuremolekül bestimmt wird, das eine Sequenz umfasst, die zu der microRNA revers komplementär ist.

6. Analytisches Verfahren nach einem der voranstehenden Ansprüche, **gekennzeichnet durch** Zugabe einer bekannten Menge einer nicht menschlichen microRNA zu der Probe vor dem Bestimmen der Konzentrationen der microRNA, Bestimmen der Konzentration der nicht menschlichen microRNA und Berechnen der Konzentration der microRNA in Bezug auf die Menge der zugesetzten nicht menschlichen microRNA.

7. Analytisches Verfahren nach einem der voranstehenden Ansprüche, **dadurch gekennzeichnet, dass** die Probe eine Blutprobe ist, bevorzugt eine Serumprobe.

8. Analytisches Verfahren nach einem der Ansprüche 2 bis 7, **dadurch gekennzeichnet, dass** die Konzentration von miR-1 im Vergleich zur Konzentration von miR-1 in einer Probe von einer gesunden Personen als um einen Faktor von etwa 2 bis 3 erhöht bestimmt wird, TTC anzeigt, und/oder dass eine Konzentration von miR-133a, die im Vergleich zur Konzentration von miR-133a in einer Probe von einer gesunden Person, als um einen Faktor von maximal 2 bis 4 erhöht bestimmt ist, TTC anzeigt, und/oder dass eine Konzentration der microRNA miR-410 (SEQ ID NO: 3) im Vergleich zur Konzentration in einer Probe von einer gesunden Person um einen Faktor von annähernd 3 erhöht bestimmt wird, TTC anzeigt, und/oder dass eine Konzentration von miR-640 (SEQ ID NO: 4), die um einen Faktor von etwa 1,5 im Vergleich zu der Konzentration in einer Probe von einer gesunden Person bestimmt wird, TTC anzeigt.

9. Analytisches Verfahren nach Anspruch 8, **dadurch gekennzeichnet, dass** die erhöhten Konzentrationen von miR-1, miR-133a, miR-410 und/oder miR-640 die Abwesenheit von STEMI anzeigen.

10. Analytisches Verfahren nach einem der voranstehenden Ansprüche, **dadurch gekennzeichnet, dass** die Konzentration der microRNA in einer Probe bestimmt wird, die zu einem ersten Zeitpunkt geNO:mmen wurde und in einer Probe, die zu einem späteren zweiten Zeitpunkt von demselben Patienten geNO:mmen wurde.

11. Anwaltliches Verfahren nach Anspruch 10, **dadurch gekennzeichnet, dass** eine Konzentration von miR-26a und/oder von miR-16, die in der Probe, die zu dem zweiten Zeitpunkt geNO:mmen wurde, im Vergleich zu der Konzentration in der Probe, die zu dem ersten Zeitpunkt geNO:mmen wurde, die verringert ist, TTC anzeigt.

12. Analytisches Verfahren nach einem der voranstehenden Ansprüche, **gekennzeichnet durch** das Zusetzen einer bekannten Menge einer microRNA, die aus der Gruppe ausgewählt ist, die aus miR-16 (SEQ ID NO: 1) und miR-26a (SEQ ID NO: 2) besteht, vor dem Bestimmen der Konzentration der microRNA zu der Probe, und wenn die Konzentration der microRNA bestimmt wird, Verringern der Konzentration der microRNA um die Menge, die vorbestimmt wurde, dass sie aus der zugesetzten microRNA erzeugt wurde.

13. Analytisches Verfahren nach einem der Ansprüche 6 bis 12, **gekennzeichnet durch** Zugabe der bekannten Menge einer nicht menschlichen microRNA und einer microRNA, die aus der Gruppe ausgewählt ist, die aus miR-16 (SEQ ID NO: 1) und miR-26a (SEQ ID NO: 2) besteht, vor dem Isolieren von RNA aus der Probe.

14. Verwendung eines Satzes von Teilen in einem Verfahren nach einem der voranstehenden Ansprüche zur Analyse einer Patientenprobe zum Nachweisen eines Analyten, der die Takotsubo Kardiomyopathie (TTC) in einer Patientenprobe anzeigt, wobei der Satz ein Primerpaar umfasst, die in wenigstens einem Endabschnitt revers komplementär zu einer der microRNAs aus der Gruppe sind, die aus miR-16 (SEQ ID NO: 1) und miR-26a (SEQ ID NO: 2) besteht.

15. Verwendung nach Anspruch 14, wobei der Satz des Weiteren eine RNA umfasst, die miR-16 (SEQ ID NO: 1) und miR-26a (SEQ ID NO: 2) umfasst oder daraus besteht, zur Verwendung als ein zu der Probe zugesetzter Standard.

## Revendications

1. Procédé d'analyse pour la détection de la cardiomyopathie de Takotsubo (CTT) dans un échantillon d'un patient en déterminant la concentration de micro-ARN dans les acides nucléiques de l'échantillon, lequel micro-ARN est choisi dans le groupe comprenant miR-16 (SEQ ID NO: 1) et miR-26a (SEQ ID NO: 2), **caractérisé en ce que** la concentration du micro-ARN choisi parmi miR-16 (SEQ ID NO: 1) et miR-26a (SEQ ID NO: 2) est détectée comme étant significativement plus élevée par rapport à la concentration du micro-ARN déterminée dans un échantillon d'une personne en bonne santé et/ou par rapport à la concentration du micro-ARN déterminée dans un échantillon d'un patient STEMI.

2. Procédé d'analyse selon la revendication 1, **caractérisé en outre en ce que** l'on détermine la concentration d'au moins un micro-ARN dans le groupe constitué par miR-1 (SEQ ID NO: 3) et/ou miR-133a (SEQ ID NO: 4).

3. Procédé d'analyse selon l'une des revendications précédentes, **caractérisé en outre en ce que** l'on détermine la concentration d'au moins un micro-ARN dans le groupe constitué par miR let-7f (SEQ ID NO: 5), miR-410 (SEQ ID NO: 6) et/ou miR-640 (SEQ ID NO: 7).

4. Procédé d'analyse selon l'une quelconque des revendications précédentes, **caractérisé en ce que** la concentration est déterminée par réaction en chaîne par polymérase de transcription inverse quantitative en utilisant des paires d'amorces spécifiques pour l'amplification du micro-ARN.

5. Procédé d'analyse selon l'une quelconque des revendications précédentes, **caractérisé en ce que** la concentration est déterminée par la détection de l'hybridation en utilisant une molécule d'acide nucléique comprenant une séquence qui est complémentaire inverse du micro-ARN.

6. Procédé d'analyse selon l'une quelconque des revendications précédentes, **caractérisé par** l'addition à l'échantillon une quantité connue d'un micro-ARN non humain avant la détermination de la concentration du micro-ARN, par la détermination de la concentration du micro-ARN non-humain et par le calcul de la concentration du micro-ARN par rapport à la quantité du micro-ARN non humain ajouté.

7. Procédé d'analyse selon l'une quelconque des revendications précédentes, **caractérisé en ce que** l'échantillon est un échantillon de sang, de préférence un échantillon de sérum.

8. Procédé d'analyse selon l'une quelconque des revendications 2 à 7, **caractérisé en ce qu'**une concentration de miR-1 déterminée comme une augmentation d'un facteur d'environ 2 à 3 par rapport à la concentration de miR-1 dans un échantillon provenant d'une personne en bonne santé est indicative de la CTT, et/ou qu'une concentration de miR-133a déterminée comme une augmentation d'un facteur d'environ 2 à 4 au maximum par rapport à la concentration de miR-133a dans un échantillon provenant d'une personne en bonne santé est indicative de la CTT et/ou qu'une concentration du micro-ARN miR-410 (SEQ ID NO : 3) déterminée comme une augmentation d'un facteur d'environ 3 par rapport à la concentration dans un échantillon provenant d'une personne en bonne santé est indicative de la CTT et/ou une concentration de miR-640 (SEQ ID NO : 4) déterminée comme une augmentation d'un facteur d'environ 1,5 par rapport à la concentration dans un échantillon provenant d'une personne en bonne santé est indicative de la CTT.

9. Procédé d'analyse selon la revendication 8, **caractérisé en ce que** les concentrations de miR-1, miR-133a, miR-410 et/ou de miR-640 sont indicatives de l'absence de STEMI.

10. Procédé d'analyse selon l'une quelconque des revendications précédentes, **caractérisé en ce que** la concentration du micro-ARN est déterminée dans un échantillon prélevé en un premier instant dans le temps et dans un échantillon prélevé à un deuxième instant ultérieur, chez le même patient.

11. Procédé d'analyse selon la revendication 10, **caractérisé en ce qu'**une concentration de miR-26a et/ou de miR-16, diminuée dans l'échantillon prélevé au deuxième instant dans le temps par rapport à la concentration dans l'échantillon prélevé au premier instant dans le temps, est indicative de la CTT.

12. Procédé d'analyse selon l'une quelconque des revendications précédentes, **caractérisé par** l'addition à l'échantillon une quantité connue d'un micro-ARN choisi dans le groupe consistant en miR-16 (SEQ ID NO : 1) et miR-26a (SEQ ID NO : 2) avant la détermination de la concentration du micro-ARN et lors de la détermination de la concentration du micro-ARN, par la réduction de la concentration du micro-ARN par rapport à la quantité prédéterminée à générer à partir du micro-ARN ajouté.

13. Procédé d'analyse selon l'une quelconque des revendications 6 à 12, **caractérisé par** l'addition de la quantité connue d'un micro-ARN non humain et/ou d'un micro-ARN choisi dans le groupe consistant en miR-16 (SEQ ID NO: 1) et miR-26a (SEQ ID NO: 2) avant l'isolement de l'ARN par rapport à l'échantillon.

14. Utilisation d'un kit de pièces dans un procédé selon l'une quelconque des revendications précédentes pour l'analyse d'un échantillon de patient pour détecter un analyte indicatif de la cardiomyopathie de Takotsubo (CTT) dans l'échantillon du patient, le kit comprenant une paire d'amorces qui sont au moins dans une section terminale inverse complémentaire de l'un des micro-ARN du groupe consistant en miR-16 (SEQ ID NO: 1) et miR-26a (SEQ ID NO: 2).

15. Utilisation selon la revendication 14, le kit comprenant en outre un ARN comprenant ou consistant en miR-16 (SEQ ID NO: 1) et miR-26a (SEQ ID NO: 2) pour utilisation comme standard ajouté à l'échantillon.
